**Europäisches Patentamt**

**European Patent Office**

**Office européen des brevets**

(19)

(11) Veröffentlichungsnummer: **0 131 847**

**B1**

(12) **EUROPÄISCHE PATENTSCHRIFT**

(45) Veröffentlichungstag der Patentschrift:
12.10.88

(21) Anmeldenummer: **84107758.9**

(22) Anmeldetag: **04.07.84**

(51) Int. Cl.⁴: **A 61 M 23/00**

(54) **Versteifungssonde.**

(30) Priorität: **15.07.83 DE 3325650**

(43) Veröffentlichungstag der Anmeldung:
**23.01.85 Patentblatt 85/4**

(45) Bekanntmachung des Hinweises auf die Patenterteilung:
**12.10.88 Patentblatt 88/41**

(84) Benannte Vertragsstaaten:
**DE FR GB**

(56) Entgegenhaltungen:
**DE-A-1 575 701**
**FR-A-977 515**
**US-A-3 349 526**

(73) Patentinhaber: **Karl Storz GmbH & Co.,
Mittelstrasse 8, D-7200 Tuttlingen (DE)**

(72) Erfinder: **Frimberger, Eckart, Tristanstrasse 12,
D-8000 München 40 (DE)**

(74) Vertreter: **Körber, Wolfhart, Dr., Patentanwälte
Dipl.- Ing. H. Mitscherlich Dipl.- Ing. K.
Gunschmann Dr.rer.nat. W. Körber Dipl.Ing. J.
Schmidt- Evers Dipl.- Ing. W. Melzer
Steinsdorfstrasse 10, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung bezieht sich auf eine Versteifungssonde nach dem Oberbegriff des Anspruchs 1.

Verschiedene Eingriffe in der Medizin können nur unter Zuhilfenahme von sogenannten Versteifungsdrähten durchgeführt werden. Dies trifft beispielsweise zu auf die Plazierung von Schläuchen in den Zwölffingerdarm, zur Ernährung oder zum Absaugen von Flüssigkeit zur Diagnostik. Ein peroral über die Speiseröhre in den Magen vorgeschobener weicher Schlauch bildet beim weiteren Vorschieben im Magen zahlreiche Schlingen, die es unmöglich machen, den Schlauch aus dem Magen hinaus in den Zwölffingerdarm vorzuschieben. Venn in den Schlauch ein rigider Draht eingeführt wird, kann der Magen ohne wesentliche Schlingenbildung passiert werden. Nachdem der Zwölffingerdarm erreicht ist, wird der Versteifungsdraht aus dem Schlauch entfernt.

Ähnliche Probleme gibt es bei der Plazierung von Speiseröhren-Prothesen und Prothesen im Gallengangbereich. Die hierzu verwendeten Versteifungsdrähte weisen jedoch den Nachteil auf, daß sie eine vorgegebene Rigidität aufweisen, was beim Passieren von Organen, wo beispielsweise ein weicherer Draht oder wiederum ein härterer Draht günstiger wäre, sich unvorteilhaft auswirkt.

Eine Versteifungssonde der eingangs bezeichneten Art ist in der US-A-3 349 526 beschrieben und dargestellt. Bei dieser bekannten Ausgestaltung wird der die Draht-Seele umgebende Mantel durch eine Mehrzahl in seiner Längsrichtung hintereinanderliegender Glieder gebildet. Bei dieser Ausgestaltung ist die Form, die die Versteifungssonde im versteiften Zustand einnimmt, durch die Anlage der Glieder aneinander vorbestimmt. Beim Versteifen der Versteifungssonde wird diese zwangsläufig die vorbestimmte Form einnehmen. In eine andere Form kann die Versteifungssonde nicht versteift werden.

Der Erfindung liegt die Aufgabe zugrunde, eine Versteifungssonde der eingangs bezeichneten Art so auszugestalten, daß sie in wahlweisen Formen versteift werden kann.

Diese Aufgabe wird durch die kennzeichnenden Merkmale des Anspruchs 1 gelöst.

Bei der erfindungsgemäßen Ausgestaltung sind die Flächen, mit denen die Glieder aneinanderliegen, sphärische Flächen. Hierdurch läßt sich die Sonde in ihrer jeweiligen Form versteifen, was insbesondere beim Einführen in empfindliche Körperteile von Bedeutung ist. Bei allen vorbeschriebenen Ausgestaltungen ist beispielsweise der Vorteil gegeben, daß beim Einführen eines Schlauches in den Zwölffingerdarm die als Einführhilfe verwendete Versteifungssonde ein verhältnismäßig angenehmes perorales Einschieben über die Speiseröhre in den Magen erlaubt. Beim Eintreten des vorderen Schlauchendes in den Magen, kann nun die Versteifungssonde entsprechend rigidisiert werden, so daß der Schlauch problemlos in den Zwölffingerdarm vorgeschoben werden kann. Mach Erreichen des Zwölffingerdarms wird die Versteifungssonde wieder entspannt, und sie kann ohne unangenehme Erscheinungen für den Patienten aus dem Schlauch entfernt werden.

Die Ausgestaltung nach Anspruch 2 umfaßt eine vorteilhafte Maßnahme zur Halterung der Glieder.

Durch die Ausgestaltung nach Anspruch 3 wird die Flexibilität der Sonde verbessert.

Die Ausgestaltungen nach Anspruch 4 empfehlen sich, um eine Spanneinrichtung einfach und schnell an die Sonde anzusetzen bzw. von ihr zu entfernen.

Das oder die Anschlagteile können je nach Bedarf die Form einer Kugel oder einer senkrecht mit ihrer Stirnfläche zur Zugrichtung angeordnete Scheibe aufweisen (Anspruch 5), sie können aber auch jede andere, zu therapeutischen oder anderen Zwecken oder zum festen Spannen in der Spanneinrichtung vorteilhafte Form aufweisen.

Gemäß Anspruch 6 können die Anschlagteile unlösbar durch Kleben, Schweißen oder ähnliches befestigt sein. Sie können aber auch beispielsweise über ein Schraubgewinde gemäß Anspruch 7 lösbar befestigt sein. Letzterer Fall weist den Vorteil auf, daß nach Ablösen des Anschlagteils die Seele aus dem Mantel herausgezogen und somit beide Teile wirksamer gereinigt und desinfiziert werden können. Des weiteren kann ein lösbar befestigtes Anschlagteil durch ein Anschlagteil anderer Form ersetzt werden, so daß die Versteifungssonde den unterschiedlichsten Verwendungsmöglichkeiten in der Medizin zugedacht werden kann.

Es ist von Vorteil, wenn beide aus dem Mantel hervorragende Enden der Seele des Bowdenzugs je ein Anschlagteil oder am einen Ende ein Anschlag- oder Befestigungsteil und am anderen Ende ein zu therapeutischen oder diagnostischen Zwecken aktives Teil aufweisen.

Bei einer Ausbildung gemäß Anspruch 8 geht das Anschlagteil nicht verloren, wenn es sich aufgrund der Spannung von der Seele lösen sollte. Außerdem bleibt die Brauchbarkeit der Versteifungssonde auch nach einem Ablösen des inneren Anschlagteils erhalten, und es werden Verletzungen vermieden.

Durch die Ausgestaltung nach Anspruch 9 kann die Entnahme von festen Proben ermöglicht werden, beispielsweise eines Gallensteins, oder diese Ausgestaltung kann sogar zum Zerquetschen eines Gallen- oder Blasensteins benutzt werden. Der Korb ist dabei derart geformt, daß zur Aufnahme des Steines das aus dem Mantel hervorgeschobene Seelenende den Korb sich radial erweitern läßt. Durch Zurückziehen der Seele wird der Korb das Besstreben zeigen, seine längs gerichteten Drähte radial einwärts zu drücken, und somit den Korb völlig zusammengelegt in das Mantel innere hineinzuzwängen. Durch diese radiale Einwärts-Druckbewegung kann ein in den Korb aufgenommener Stein verklemmt und aus dem Körper entfernt oder sogar derart zerquetscht werden, daß die feinen Steinfragmente aus dem Organ ausgeschieden werden können.

Gemäß Anspruch 10 kann eine glatte Oberfläche erreicht werden.

So können bei einer Ausbildung gemäß Anspruch 11 Verunreinigungen vermieden und die Sauberhaltung des Gerätes besser vollzogen werden. Auch kann ein entsprechender Kunststoff- oder sonst in der Medizin gebräuchlicher Überzug auf allen Oberflächen der erfindungsgemäßen Sonde vorgesehen sein.

Des weiteren kann die erfindungsgemäße Sonde an dem aus dem Mantel hervorragenden vorderen Ende ein über das Anschlagteil herausragendes weiches Führungsende aufweisen, was das Einführen der Sonde erleichtert.

Dem gleichen Zweck dient auch die Ausgestaltung nach Anspruch 12, wobei gleichzeitig ein leichtes und bequemes Anschließen einer Spanneinrichtung oder ein leichtes und bequemes Bewegungsblockieren von Seele und Mantel mittels einer Arretierungseinrichtung ermöglicht ist.

Im Anspruch 13 sind Ausgestaltungsmerkmale enthalten, die eine Bewegungsblockierung zwischen einer Sonde und einem darauf aufzuschiebenden Schubglied ermöglichen. Hierzu kann vorteilhaft ein entweder auf dem Schubglied oder auf der Sonde befestigtes Klemmteil oder Kupplungsteil dienen, das mit einem jeweils am anderen Teil befestigten Kupplungsteil zusammenwirkt. Durch diese Ausgestaltung wird die Handhabung der Sonde beim Einführen einer Prothese oder dgl. erheblich vereinfacht.

Nachfolgend wird die Erfindung anhand mehrerer, in einer Zeichnung dargestellter Ausführungsbeispiele näher beschrieben. Es zeigt:

Fig.1 eine erfindungsgemäße Versteifungs-Sonde im axialen Schnitt;
Fig. 2 bis 5 weitere Ausführungsbeispiele der Sonde.

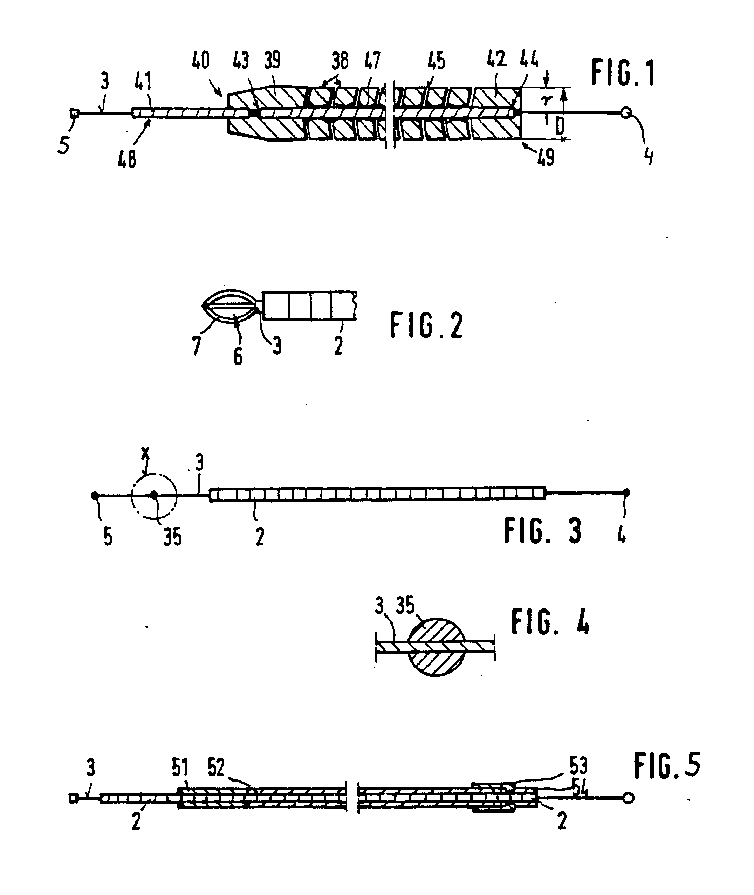

Wie aus Fig. 1 zu entnehmen ist, besteht die erfindungsgemäße Versteifungs-Sonde aus einem Mantel 40 und einer in diesem axial verschieblich angeordneten weichen Seele 3. An den beiden aus dem Mantel 40 hervorragenden Enden der Seele 3 sind jeweils Anschläge befestigt, und zwar beispielhaft in dieser Ausführung ein kugelförmiger Anschlag 4 und am anderen Ende ein scheibenförmiger Anschlag 5.

Wie aus Fig. 2 zu ersehen ist, kann der Anschlagteil auch ein aus längsgerichteten elastischen Federstahldrähten 7 geformter Korb 6 sein.

Der Außendurchmesser einer Sonde ist dadurch limitiert, daß einerseits die Seele 3 einen Durchmesser von etwa 1 bis 1,5 mm nicht wesentlich überschreiten darf, da sie sonst zu steif wird, was Schwierigkeiten beim Einführen in eine Stenose mit sich bringt. Außerdem darf insbesondere bei einem aus Wicklungen bestehenden Mantel 2 das Spiel zwischen der Innenwand des Mantels 2 und der Seele 3 nur gering sein, da sich sonst beim Spannen der Sonde der Mantel 2 wellenförmig verformt und bei einem aus Wicklungen bestehenden Mantel 2 sich einzelne Wicklungen übereinanderschieben. Bei einem solchen Ausführungsbeispiel kann auch die Wandstärke des Mantels 2 nicht frei vergrößert werden, da bei einer Stärke der Wicklungen von mehr als 0,6 bis 0,8 mm der Mantel 2 zu steif wird.

Wie aus Fig. 1 zu entnehmen ist, besteht der Mantel 40 aus in seiner Längsrichtung hintereinanderliegenden ringförmigen Gliedern 38, deren Flächen 45, mit denen sie aneinanderliegen, sphärische Flächen sind, und die beweglich aneinander gehalten sind. Diese Halterung der Glieder 38, aneinander kann auf verschiedene Weise, z. B. durch eine für alle Glieder 38 gemeinsame Außenhülle (flexibler Schlauch) gebildet werden, die zugleich die Glieder 38 und die zwischen ihnen bestehenden Spalte vor Verschmutzung schützt. Von Bedeutung ist, daß die Seele 3 entweder mit dem ersten Glied 39 in Verbindung steht oder mit dem Anschlagteil 5 mittelbar oder unmittelbar gegen das erste Glied 39 ziehbar ist, um die Versteifung zu verwirklichen. Die Versteifung erfolgt durch die Spannung, die bei gegenseitiger Anlage der Glieder 38 besteht. Dabei brauchen die Glieder 38 nicht ganzflächig aneinander anzuliegen, sondern eine Versteifung ist schon bei punktförmiger Anlage erreicht, bei der die Sonde eine von einer Geraden abweichende Form einnehmen kann. Aufgrund der sphärischen Flächen der Glieder 38 ist eine stabile Versteifung auch in einer von einer geraden abweichenden Form der Sonde möglich, beispielsweise einfach oder S-förmig gekrümmt.

Bei den zu beschreibenden Ausführungsbeispielen ist der allgemein mit 40 bezeichnete Mantel der Sonde durch einen Kernmantel 41 gebildet, der aus homogenem Material (beispielsweise aus Kunststoff) oder aus aneinanderliegenden Wicklungen besteht und auf dem die ringförmigen Glieder 38 aufgezogen und aneinander gehalten sind. Das erste Glied 39 und das letzte Glied 42 sind starr, beispielsweise durch Löten, auf dem Kernmantel 41 befestigt. Zum Zweck der Erhöhung der Festigkeit hintergreifen das erste Glied 39 und das letzte Glied 42 den inneren Abschnitt des Kernmantels 41 formschlüssig an den mit 43 und 44 bezeichneten Stellen. An diesen Stellen 43, 44 ist der Kernmantel 41 unterbrochen bzw. verkürzt, wobei die Glieder 39, 42 mit Vorsprüngen die vorhandenen Stirnflächen des Kernmantels 41 hinterfassen. Ein Stück vorbestimmter Länge des Kernmantels 41 ragt einführseitig aus dem Glied 39 hervor, womit das Einführen erleichtert wird. Ebenfalls zur erleichterten Einführung ist das erste Glied 39 jeweils konisch zugespitzt. Beim vorliegenden Ausführungsbeispiel steht nicht nur ein großer Sondendurchmesser D zur Verfügung, sondern es kann auch eine größere Versteifung erreicht werden, weil größere Radien r vorliegen, von deren Größe das Maß der Versteifung abhängig ist.

Die Anschlagkanten 43, 44 können auch fehlen, wenn das erste Glied 39 und das letzte Glied 42 durch Löten auf dem aus Wicklungen bestehenden Metallmantel 2 befestigt sind.

Um die Flexibilität zu vergrößern, ist es vorteilhaft, ein Spiel in den Bohrungen 47 für den Kernmantel 41 vorzusehen. Es ist der Flexibilität insbesondere zuträglich, wenn gemäß Fig. 1 die Bohrungen 47 sich nach außen jeweils erweitern.

Zum Spannen der Sonde wird das dem Einführende 48 abgewandte Ende 49 des Mantels 40 in eine Halterung einer nicht dargestellten Spanneinrichtung aufgenommen.

Beim Ausführungsbeispiel gemäß Fig. 3 und 4 besteht die Sonde ebenfalls aus einem allgemein mit 2 bezeichneten Mantel und einer darin eingeschobenen Seele 3 mit einem vorderen Anschlagteil 5 und einem hinteren Anschlagteil 4 in Kugelform. Darüber hinaus ist in einem Abstand vom Anschlagteil 5 ein weiteres Anschlagteil 35 auf der Seele 3 angeordnet. Der Zweck des Anschlagteils 35 ist folgender.

Löst sich beim Spannen der Sonde aufgrund der Spannkraft das Anschlagteil am vorderen Ende der Seele 3, z. B. aufgrund einer mangelhaften Lötstelle, wird es durch die Vorspannung des Mantels 2 mit Vehemenz weggeschleudert, was theoretisch zu Verletzungen führen könnte. Bei diesem Ausführungsbeispiel wird nicht das Anschlagteil 5, sondern das Anschlagteil 35 beim Spannen belastet, das beim Spannen am Mantel 2 zur Anlage kommt. Löst sich das Anschlagteil 35 beim Spannen unbeabsichtigt, geht es nicht verloren, sondern es wird durch das Anschlagteil 5 gehalten. Außerdem bleibt die Sonde noch funktionsfähig, weil aufgrund des Vorhandenseins des Anschlagteils 5 die Sonde weiterhin versteift werden kann.

Fig. 4 zeigt die mit X gekennzeichnete Einzelheit in Fig. 3 in vergrößerter Darstellung im Schnitt. Das Anschlagteil 35 ist eine Kugel mit einer Bohrung, die auf die Seele 3 aufgeschoben und verlötet ist.

Im Rahmen der Erfindung ist es auch möglich, mehrere zusätzliche Anschlagteile 35 vorzusehen.

Das Ausführungsbeispiel gemäß Fig. 5 zeigt eine erfindungsgemäß ausgestaltete Sonde, die zum Einführen einer Gallengangprothese 51 in Form eines Kunststoffschlauches vorbereitet ist. Die Prothese 51 ist am Einführende der Sonde auf den Mantel 2 aufgeschoben. Hinter der Prothese 51 befindet sich ein Schubglied 52 in Form eines flexiblen Schlauches, der dazu dient, in der eingeführten Position der Sonde die Prothese 51 in die gewünschte Position zu schieben.

Auf dem hinteren Ende des Schubgliedes 52 ist ein erstes Kupplungsglied 53 befestigt, das an ein zweites Kupplungsglied 54 angekuppelt ist, das auf dem Mantel 2 befestigt ist. In bevorzugter Ausführungsform sind die Kupplungsglieder 53, 54 in der Medizintechnik bekannte Luerlock-Anschlußteile, die sich sehr gut eignen.

Es ist der Zweck der Kupplungsglieder 53, 54 eine Fixierung zwischen der Sonde (Mantel 2) und dem Schubglied 52 herbeizuführen, so daß eine Relativverschiebung zwischen diesen Teilen nicht erfolgen kann.

Eine Relativverschiebung kann beim Einführen einer Sonde erfolgen, wenn das Einführende beim Einführen auf Widerstand stößt, der die Sonde im Schubglied 52 auswärts zu verschieben sucht. Außerdem führt das Versteifen der Sonde zu einer Kraftkomponente, die dann die Sonde auswärts beaufschlagt, wenn sie in einer eingeführten Position in gekrümmter Stellung versteift wird. Schon durch die Tendenz einer Relativverschiebung wird der behandelnde Arzt erheblich behindert. Eine stattgefundene Relativverschiebung kann sogar zu einem Mißlingen des Eingriffs führen, weil die Prothese 51 eine unpassende Position einnimmt.

Zur Vorbereitung der Sonde wird zunächst das Schubglied 52 vom Einführungsende her aufgeschoben und mit Hilfe der Kupplungsglieder 53, 54 an der Sonde festgelegt. Anschließend wird die Prothese 51 vom Einführende her aufgeschoben. Um die Position der Prothese 51 auf der Sonde einstellen zu können, ist es vorteilhaft das Kupplungsglied 54 verstellbar und in der jeweiligen gewünschten Position feststellbar auf der Sonde, im vorliegenden Falle auf dem Mantel 2, anzuordnen. Hierzu kann beispielsweise eine Klemmschraube dienen.

Die Maßnahme, eine Prothese 51 und das Schubglied 52 auf eine eingeführte Sonde vom hinteren Ende her aufzuschieben ist nachteilig. Zum einen deshalb, weil es bei einem Schubglied 52 von etwa 1,5 m Lange einer schwierigen Handhabung bedarf, zum anderen deshalb, weil die Position der eingeführten Sonde nur dann kontrolliert werden kann, wenn sie immer mit ihrem hinteren Ende aus dem Schubglied 52 herausragt, also etwa 1,5 m länger als an sich erforderlich, bemessen ist. Eine solch lange Sonde ist unhandlich.

**Patentansprüche**

1. Versteifungs-Sonde (1) zur Verwendung in der Medizin, beispielsweise zu therapeutischen oder diagnostischen Zwecken, insbesondere zur Einführung in den Verdauungs- oder Ausscheidungtrakt, mit drahtförmiger Ausbildung, wobei die Versteifungs-Sonde (1) durch einen aus einem Mantel (2, 40, 41) und einer Draht-Seele (3) bestehenden Bowdenzug gebildet ist und die Draht-Seele (3) am Einführende ein Anschlagteil (5) aufweist, das bei Zug an der Draht-Seele (3) am dem Einführende abgewandten Ende am Mantel (2, 40, 41) oder Anbauteilen desselben zur Anlage kommt, und wobei der Mantel (2) in seiner Längsrichtung hintereinanderliegende Glieder (38) aufweist, die beweglich aneinander gehalten sind,
    dadurch gekennzeichnet,
    daß die Flächen (45), mit denen die Glieder (38) aneinanderliegen, sphärische Flächen (45) sind.
    2. Sonde nach Anspruch 1,
    dadurch gekennzeichnet,
    daß die Glieder (38) auf einem Kernmantel (41) aufgezogen und gehalten sind.
    3. Sonde nach Anspruch 2,
    dadurch gekennzeichnet,
    daß die den Kernmantel (41) aufnehmenden Bohrungen (47) in den Gliedern (38) sich nach außen jeweils erweitern.
    4. Sonde nach einem der Ansprüche 1 bis 3,
    dadurch gekennzeichnet,
    daß die aus dem Mantel (2, 40) bzw. Kernmantel (41) herausragende Seele (3) des Bowdenzuges am dem Einführende abgewandten Ende ein Anschlagteil (4) aufweist.
    5. Sonde nach einem der Ansprüche 1 bis 4,
    dadurch gekennzeichnet,
    daß die Anschlagteile (4, 5) kugelförmige (4) oder scheibenförmige (5) Teile sind.
    6. Sonde nach einem der Ansprüche 1 bis 5,
    dadurch gekennzeichnet,
    daß das Anschlagteil (4, 5) am Ende der Seele (3) in an sich bekannter Weise unlösbar befestigt ist.
    7. Sonde nach Anspruch 6,
    dadurch gekennzeichnet,
    daß das Anschlagteil (4, 5) am Ende der Seele (3) beispielsweise über ein Schraubgewinde lösbar befestigt ist.
    8. Sonde nach einem der Ansprüche 1 bis 7,
    dadurch gekennzeichnet,
    daß die Seele (3) am Einführende zwei hintereinander liegende Anschlagteile (5, 35) aufweist.
    9. Sonde nach einem der Ansprüche 1 bis 7,
    dadurch gekennzeichnet,
    daß das Anschlagteil am Einführende der Seele (3) ein aus längsgerichteten Federstahldrähten (7) geformter, axial gestreckter Korb (6) ist.
    10. Sonde nach einem der Ansprüche 1 bis 9,
    dadurch gekennzeichnet,
    daß zumindest die äußere Oberfläche des Mantels (2) eine glatte Kunststoffbeschichtung aufweist.
    11. Sonde nach einem der Ansprüche 1 bis 10,
    dadurch gekennzeichnet,
    daß die Glieder (38) in einer gemeinsamen flexiblen Außenhülle aufgenommen sind.
    12. Sonde nach einem der Ansprüche 1 bis 11,
    dadurch gekennzeichnet,
    daß zu ihrer leichteren Einführbarkeit die Seele (3) zu beiden Seiten des Mantels (2) in vorbestimmter Weise hervorragt, wobei das am hinteren Ende der Sonde (1) hervorragende Ende der Seele (3) längenmäßig derart dimensioniert ist, daß eine Arretierungseinrichtung zum Bewegungsblockieren von Seele zu Mantel appliziert werden kann, während das am Einführende der Sonde (1) hervorragende Ende der Seele (3) die notwendige elastische Einführlänge aufweist.
    13. Sonde nach einem der Ansprüche 1 bis 10,
    dadurch gekennzeichnet,
    daß ihr zum Zweck des Einführens von rohrförmigen Zusatzteilen, z. B. Prothesen, ein rohrförmiges, aufschiebbares Schubglied zugeordnet ist, und daß das Schubglied (52) an seinem dem Einführende abgewandten Ende ein Klemmteil oder ein Kupplungsteil (53) zur lösbaren Befestigung an der Sonde (1) aufweist.

**Claims**

    1. A stiffening probe (1) of a wire-like form, for medical use, for example for therapeutic or diagnostic purposes, more particularly for insertion into the digestive or excretory tract, the stiffening probe (1) comprising a Bowden cable concisting of a casing (2, 40, 41) and a wire core (3), the wire core (3) having at the insertion end a stop component (5) which, when the wire core (3) is pulled at the end remote from the insertion end, comes to bear on the casing (2, 40, 41) or attachment parts thereof, and the casing (2) having sections (38) lying one behind the other in its longitudinal direction which are held movably together, characterized in that the adjacent faced (45) of the sections (38) are spherical faced (45).
    2. A probe according to claim 1, characterized in that the sections (38) are drawn to and held on a core casing (41).
    3. A probe according to claim 2, characterized in that the bores (47) in the sections (38) accommodating the core casing (41) widen outwardly in each case.
    4. A probe according to any one of claims 1 to 3, characterized in that the core (3) of the Bowden cable projecting from the casing (2, 40) or the core casing (41) has a stop component (4) at the end remote from the insertion end.
    5. A probe according to any one of claims 1 to 4, charactericed in that the stop components (4, 5) are spherical (4) or discoid (5) components.
    6. A probe according to any one of claims 1 to 5, characterised in that in a way known per se the stop component (4, 5) at the end of the core (3) is fixed undetachably.
    7. A probe according to claim 6, characterized in that the stop component (4, 5) at the end of the core (3) is fixed detachably, for example by means of a screw thread.
    8. A probe according to any one of claims 1 to 7, characterized in that at the insertion end the core (3) has two stop components (5, 35) lying one behind the other.
    9. A probe according to any one of claims 1 to 7, characterised in that the stop component at the insertion end of the core (3) is an axially extending cage (6) formed by longitudinally directed spring steel wired (7).

10. A probe according to any one of claims 1 to 9, characterised in that at least the outer surface of the casing (2) has a coating of smooth plastics material.

11. A probe according to any one of claims 1 to 10, characterized in that the sections (38) are accommodated in a common flexible external jacket.

12. A probe according to any one of claims 1 to 11, characterized in that in order to make it easier to insert, the core (3) projects in a predetermined manner from both ends of the casing (2), the end of the core (3) projecting at the rear end of the probe (1) having longitudinal dimensions much that a locking device can be applied to block the movement of the core relatively to the casing, while the end of the core (3) projecting at the insertion end of the probe (1) has the necessary elastic insertion length.

13. A probe according to any one of claims 1 to 10, characterized in that for the purpose of the insertion of tubular parts e.g. prostheses, a tubular, slip-on propelling member is associated with it and that the propelling member (52) has at its end remote from the insertion end a clamp component or a coupling component (53) for detachable attachment to the probe (1).

**Revendications**

1. Sonde de raidissement (1) destinée à être utilisée en médecine, par exemple dans un but thérapeutique ou pour un diagnostic, en particulier pour l'introduction dans le tube digestif ou dans un conduit excréteur, comportant une conformation de fil, la sonde de raidissement (1) étant constitué par un système Bowden composé d'une enveloppe (2, 40, 41) et d'une âme en fil (3), et l'âme en fil (3) présentant à l'extrémité d'introduction une pièce de butée (5), qui lorsque l'âme en fil (3) est tirée vient en butée contre l'enveloppe (2, 40, 41) à l'extrémité opposée à l'extrémité d'introduction ou sur des pièces montées sur celle-ci, et l'enveloppe (2) présentant des éléments disposés les uns derrière les autres qui sont maintenus les uns contre les autres de façon à pouvoir se déplacer, caractérisée par le fait que les surfaces (45) par lesquelles les éléments sont placés les uns contre les autres, sont des surfaces sphériques (45).

2. Sonde selon la revendication 1, caractérisée par le fait que les éléments (38) sont montés sur une enveloppe centrale (41) et maintenus par celle-ci.

3. Sonde selon la revendication 2, caractérisée par le fait que les alésages (47) recevant l'enveloppe centrale (41) pratiqués dans les éléments (38) s'élargissent dans chaque cas vers l'extérieur.

4. Sonde selon une des revendications 1 à 3, caractérisée par le fait que l'âme (3) du système Bowden qui dépasse de l'enveloppe (2, 40), respectivement de l'enveloppe centrale (41), présente une pièce de butée (4) à l'extrémité opposée à l'extrémité d'introduction.

5. Sonde selon une des revendications 1 à 4, caractérisée par le fait que les pièces de butée (4, 5) sont des pièces en forme de billes (4) ou de disques (5).

6. Sonde selon une des revendications 1 à 5, caractérisée par le fait que la pièce de butée (4, 5) située à l'extrémité de l'âme (3) est fixée de façon indémontable d'une manière connue en elle-même.

7. Sonde selon la revendication 6, caractérisée par le fait que la pièce de butée (4, 5), située à l'extrémité de l'âme (3), est fixée de façon démontable par exemple au moyen d'un filetage.

8. Sonde selon une des revendications 1 à 7 caractérisée par le fait que l'âme (3) présente, à l'extrémité d'introduction, deux pièces de butée (5, 35) disposées l'une derrière l'autre.

9. Sonde selon une des revendications 1 à 7, caractérisée par le fait que la pièce de butée, située à l'extrémité d'introduction de l'âme (3), est un panier (6) s'étendant axialement et formé par des fils d'acier à ressort (7) disposés longitudinalement.

10. Sonde selon une des revendications 1 à 9, caractérisée par le fait qu'au moins la surface extérieure de l'enveloppe (2) présente une enduction de matière plastique lisse.

11. Sonde selon l'une des revendications 1 à 10, caractérisée par le fait que les éléments (38) sont logés dans une enveloppe extérieure souple commune.

12. Sonde selon une des revendications 1 à 11, caractérisée par le fait qu'en vue de s'introduire plus facilement l'âme (3) dépasse des deux côtés de l'enveloppe (2) d'une manière déterminée à l'avance, l'extrémité de l'âme (3), qui dépasse à l'extrémité arrière de la sonde (1), étant dimensionnée en longueur de façon telle que l'on peut appliquer un dispositif de blocage destiné à bloquer le mouvement de l'âme par rapport à l'enveloppe, tandis que l'extrémité de l'âme (3), qui dépasse à l'extrémité d'introduction de la sonde (1), présente la longueur d'introduction élastique nécessaire.

13. Sonde selon une des revendications 1 à 10, caractérisée par le fait qu'un élément de poussée de forme tubulaire, destiné à l'introduction de pièces accessoires de forme tubulaire, par exemple des prothèses, lui est affecté et que l'élément de poussée (52) présente, à son extrémité opposée à l'extrémité d'introduction, une pièce de blocage ou une pièce d'accouplement (53) destinée à la fixer de façon démontable sur la sonde (1).

FIG.1

FIG.2

FIG. 3

FIG. 4

FIG.5